(19) **Europäisches Patentamt / European Patent Office / Office européen des brevets**

(11) **EP 4 260 326 B1**

(12) **EUROPEAN PATENT SPECIFICATION**

(45) Date of publication and mention
of the grant of the patent:
**01.04.2026 Bulletin 2026/14**

(21) Application number: **22700895.0**

(22) Date of filing: **07.01.2022**

(51) International Patent Classification (IPC):
**G16C 20/30** *(2019.01)*

(52) Cooperative Patent Classification (CPC):
**G16C 20/30;** G16C 10/00; G16C 20/70

(86) International application number:
**PCT/EP2022/050287**

(87) International publication number:
**WO 2022/148847 (14.07.2022 Gazette 2022/28)**

(54) **PREDICTING EXCHANGE-CORRELATION ENERGIES OF ATOMIC SYSTEMS USING NEURAL NETWORKS**

VORHERSAGE VON AUSTAUSCH-KORRELATIONSENERGIEN VON ATOMSYSTEMEN UNTER VERWENDUNG NEURONALER NETZWERKE

PRÉDICTION D'ÉNERGIES D'ÉCHANGE-CORRÉLATION DE SYSTÈMES ATOMIQUES À L'AIDE DE RÉSEAUX NEURONAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.01.2021 US 202163135223 P**

(43) Date of publication of application:
**18.10.2023 Bulletin 2023/42**

(73) Proprietor: **GDM Holding LLC Mountain View, CA 94043 (US)**

(72) Inventors:
- **KIRKPATRICK, James** London N1C 4AG (GB)
- **MCMORROW, Brendan Charles** London N1C 4AG (GB)
- **TURBAN, David Herbert Phillip** London N1C 4AG (GB)
- **GAUNT, Alexander Lloyd** London N1C 4AG (GB)
- **SPENCER, James** London N1C 4AG (GB)
- **MATTHEWS, Alexander Graeme de Garis** London N1C 4AG (GB)
- **COHEN, Aron Jonathan** London N1C 4AG (GB)

(74) Representative: **Marks & Clerk GST 1 New York Street Manchester M1 4HD (GB)**

(56) References cited:
- **KIRKPATRICK JAMES ET AL: "Pushing the frontiers of density functionals by solving the fractional electron problem", SCIENCE, vol. 374, no. 6573, 10 December 2021 (2021-12-10), US, pages 1385 - 1389, XP093327505, ISSN: 0036-8075, DOI: 10.1126/science.abj6511**
- **JONATHAN SCHMIDT ET AL: "Machine Learning the Physical Non-Local Exchange-Correlation Functional of Density-Functional Theory", ARXIV.ORG, CORNELL UNIVERSITY LIBRARY, 201 OLIN LIBRARY CORNELL UNIVERSITY ITHACA, NY 14853, 17 August 2019 (2019-08-17), XP081501279**
- **RYO NAGAI ET AL: "Completing density functional theory by machine-learning hidden messages from molecules", ARXIV.ORG, CORNELL UNIVERSITY LIBRARY, 201 OLIN LIBRARY CORNELL UNIVERSITY ITHACA, NY 14853, 1 March 2019 (2019-03-01), XP081499163**

- **SEBASTIAN DICK ET AL: "Using differentiable programming to obtain an energy and density-optimized exchange-correlation functional", ARXIV.ORG, CORNELL UNIVERSITY LIBRARY, 201 OLIN LIBRARY CORNELL UNIVERSITY ITHACA, NY 14853, 8 June 2021 (2021-06-08), XP081986410**

**Description**

CROSS-REFERENCE TO RELATED APPLICATION

This application claims priority to U. S. Provisional Patent Application No. 63/135,223, on January 8, 2021.

BACKGROUND

This specification generally relates to predicting properties of atomic systems (such as a neutral or charged molecule or a neutral or charged single atom) using neural networks.

Neural networks are machine learning models that employ one or more layers of nonlinear units to predict an output for a received input. Some neural networks include one or more hidden layers in addition to an output layer. The output of each hidden layer is used as input to the next layer in the network, i.e., the next hidden layer or the output layer. Each layer of the network generates an output from a received input in accordance with current values of a respective set of parameters. J. Schmidt et al., "Machine Learning the Physical Local Exchange-Correlation Functional of Density-Functional theory", 2019, describes training a neural network as the universal exchange-correlation functional of density-functional theory. R. Nagai et al, "Completing Density Functional Theory by Machine-learning hidden messages from molecules", 2019 proposes systematic construction of a functional using accurate density distributions and energies in reference molecules via machine learning.

SUMMARY

The invention is defined in the appended claims. This specification describes methods, computer systems, and apparatus, including computer programs encoded on computer storage media, for predicting an exchange-correlation energy of an atomic system and as defined by the appended claims. In particular, the techniques include using a neural network to process electron-orbital features of the atomic system to output a predicted exchange-correlation energy of the atomic system.

In a first aspect, the invention is a method performed by one or more computers and for predicting an exchange-correlation energy of an atomic system, the method comprising:obtaining respective electron-orbital features of the atomic system at each of a plurality of grid points;generating, for each of the plurality of grid points, a respective input feature vector for the electron-orbital features at the grid point;processing the respective input feature vectors for the plurality of grid points using a neural network to generate a predicted exchange-correlation energy of the atomic system; andwherein the neural network is trained on training data that includes a plurality of training examples, the training examples each corresponding to a respective atomic system, and the training examples including a first subset of training examples that correspond to atomic systems that have electron-orbital features and energy levels satisfying one or more mathematical constraint conditions, the one or more mathematical constraint conditions including: a uniform electron gas (UEG) constraint condition, a fractional charge (FC) constraint condition, or a fractional spin (FS) constraint condition;and either:one or more of the electron-orbital features of the atomic system are obtained based on real-world measurement data; orthe method further comprises determining based on the predicted exchange-correlation energy of the atomic system, whether to perform a fabrication process of a chemical product which comprises the atomic system, and, if the determination is positive, causing the fabrication to be performed.

Each training example may be associated with a respective dataset employed in the training, and containing data describing one or more physical properties of the corresponding atomic system, such as the electron-orbital features, the energy labels and/or a molecular geometry of the atomic system (i.e. the 3D relative positions of the nuclei of the atomic system). For some of the training examples, at least some data of the dataset may have been measured in the real world (i.e. by measurements (experiments) carried out on the real-world atomic system). The method may include a preliminary step of receiving at least some of this measured data and/or a preliminary step of measuring at least some of this data (i.e. by performing a real-world experiment). Alternatively, some or all of the data may be obtained computationally.

In some implementations of the prediction method, the atomic systems corresponding to the plurality of training examples include synthetically generated virtual atomic systems with electron-orbital features and energy levels satisfying the one or more mathematical constraint conditions.

In some implementations of the prediction method, the plurality of grid points include grid points on a real-space quadrature grid.

In some implementations of the prediction method, the electron-orbital features obtained for the atomic system include one or more of: an electron density distribution, an electron density gradient norm distribution, a kinetic energy density distribution, a local Hartree-Fock (HF) exchange distribution, or a range-separated form of the local HF exchange distribution of the atomic system.

In some implementations of the prediction method, the system generates the input feature vector for each of the plurality of grid points by: converting the electron-orbital features at the grid point from a linear scale to a logarithm scale; and concatenating the electron-orbital features in the logarithm scale to form the input feature vector for the grid point.

In some implementations of the prediction method, the neural network includes: a multilayer perceptron (MLP) configured to process each of the input feature vectors at the plurality of grid points to generate a plurality of enhancement factors characterizing a plurality of contribution terms corresponding to the input feature vector; and a numerical quadrature layer configured to integrate a weighted sum of the plurality of contribution terms scaled by the enhancement factors over the plurality of grid points to generate the predicted exchange-correlation energy of the atomic system.

In some implementations of the prediction method, the plurality of contribution terms include one or more of: a local-density approximation (LDA) exchange term, an HF term, or a range-separated HF term.

In a second aspect, the invention is a computer-implemented method for training the neural network of the first aspect, the neural network having a plurality of parameters, and the method comprising: obtaining a plurality of training examples, each training example including electron-orbital features and corresponding ground-truth energy label of an atomic system, the plurality of training examples including a first subset of training examples that correspond to atomic systems that have electron-orbital features and energy levels satisfying one or more mathematical constraint conditions, wherein the one or more mathematical constraint conditions include: a uniform electron gas (UEG) constraint condition, a fractional charge (FC) constraint condition, or a fractional spin (FS) constraint condition; for each training example, processing the electron-orbital features in the training example using the neural network and in accordance with current values of the parameters to generate a predicted exchange-correlation energy for the training example; determining a gradient with respect to the parameters of a training loss, the training loss including a regression loss that measures, for each training example, an error between the predicted exchange-correlation energy for the training example and the ground-truth energy label in the training example; and updating the current values of the parameters using the gradient.

In some implementations of the training method, the one or more mathematical constraint conditions include a fractional charge (FC) constraint condition and a fractional spin (FS) constraint condition.

In some implementations of the training method, the training loss further includes a self-consistent field (SCF) loss, the SCF loss representing a calculated energy of the atomic system subject to electron number conservation.

In some implementations of the training method, the system obtains the first subset of training examples by numerically synthesizing virtual atomic systems with electron-orbital features and energy levels satisfying the one or more mathematical constraint conditions.

In a third aspect, the invention is a system according to claim 14 including one or more computers and one or more storage devices storing instructions that when executed by the one or more computers, cause the one or more computers to perform the prediction method and/or the training method described above.

In a fourth aspect, the invention provides one or more computer storage media according to claim 15 storing instructions that when executed by one or more computers, cause the one or more computers to perform the prediction method and/or the training method described above.

The subject matter described in this specification can be implemented in particular embodiments so as to realize one or more of the following advantages.

Density functional theory (DFT) revolutionized quantum chemistry as the only computational method to promise high accuracy and to scale to large chemical systems. Key to its success is the efficient description of the quantum-mechanical interaction between electrons by approximation of the exact exchange-correlation functional, $E_{xc}$. In trying to satisfy the accuracy and broad applicability demanded in diverse scientific fields, a large range of such approximations has evolved. However, fundamental failings persist in modern general-purpose functionals, which impact broad areas of chemistry and are even evident on molecules as simple as $H_2^+$. Failures can often be traced back to the violation of mathematical properties of the exact functional, for example, the violation of exact conditions for systems exhibiting behaviors of fractional electrons.

The described techniques use deep learning to produce functionals (e. g., the exchange-correlation functional, $E_{xc}$) that achieve state-of-the-art performance on large general chemistry databases, and that also allow incorporation of exact constraints in a data-driven manner. Notably, the described techniques produce accurate functionals that solve the problem of simultaneously obeying the known constraints for systems with fractional electron charge and spin. The functionals produced by the described techniques can be used to accurately predict the energy of atomic systems in the DFT framework. The predicted energies of the atomic systems can be used to predict important chemical properties, such as the ionization energies, the vibrational properties, the enthalpies of formation, and the reaction barriers of the corresponding atomic systems.

The described techniques allow behavior learned from training on these fictional atomic systems to generalize to larger real molecules. The constraints are demonstrated to contribute to superior approximations of the exchange-correlation energies of a broad range of atomic systems, and thus surpassing state-of-the-art for predicting properties of atomic systems using DFT.

Based on the predicted exchange-correlation energy of the atomic system, e.g., a reactant for a chemical reaction, the system can obtain properties such as the ionization energy, the vibrational properties, the enthalpies of formation, and the reaction barriers of the reactant. Based on these properties, the system can make a decision for producing a chemical product (e.g., a drug molecule) using the reactant. For example, the system can generate an output that indicates whether the chemical reaction can take place under a specified condition to produce a chemical product, or an output that specifies the optimal reaction parameters for producing the chemical product. The system can transmit the output to a fabrication apparatus operative to implement the instruction to produce the chemical product.

The details of one or more embodiments of the subject matter described in this specification are set forth in the accompanying drawings and the description below. Other features, aspects, and advantages of the subject matter will become apparent from the description, the drawings, and the claims.

BRIEF DESCRIPTION OF THE DRAWINGS

FIG. 1 shows an example of an exchange-correlation energy prediction system.

FIG. 2 illustrates examples of predicting an exchange-correlation energy of an atomic system and training a neural network for predicting an exchange-correlation energy.

FIG. 3 is a flow diagram illustrating an example process for predicting exchange-correlation energy using neural networks.

FIG. 4 is a flow diagram illustrating an example process for training a neural network for predicting an exchange-correlation energy of an atomic system.

Like reference numbers and designations in the various drawings indicate like elements.

DETAILED DESCRIPTION

Computing electronic energies underpin theoretical chemistry and materials science, and density functional theory (DFT) is an exact and efficient approach. The power of DFT is that it only deals with the simple electron density, rather than the exponentially scaling many-electron wavefunction, while retaining theoretical guarantees that exact ground-state properties are computable.

For example, in the Kohn-Sham (KS) formulation of DFT, the ground state energy E depends on single electron orbitals $\phi_i$, which are used to compute the electron density $\rho$ and the kinetic energy $T_s[\rho]$, and also on the external potential v and Coulomb energy $J[\rho]$. Additional remaining intricate electronic effects are grouped in an unknown universal exchange-correlation functional, $E_{xc}[\rho]$:

$$E[\rho] = T_s[\rho] + \int \rho(\mathbf{r})v(\mathbf{r})\mathrm{d}\mathbf{r} + J[\rho] + E_{xc}[\rho] \qquad (1)$$

Although the exact functional-mapping electron density to energies, e.g., $E_{xc}[\rho]$, has been proven to exist, little practical guidance is given on its explicit form. Approximations to the exact functional have enabled numerous investigations of matter at a microscopic level. However, errors persist in those approximations. It has been known that the accuracy and application range of the approximations improve when they satisfy more of the mathematical constraints of the exact functional, and the root cause of many of the approximation errors is the violation of exact conditions for systems with fractional electrons.

The techniques described in this specification include generating a functional that is trained to satisfy one or more specified conditions (e.g., exact conditions for systems with fractional electrons), and thus provide improved performance for predicting behaviors of atomic systems across main-group chemistry.

In particular, the techniques may be used to obtain a ground-state energy for the atomic system. This information allows a decision to be made about whether to perform a process in the real world, such as the fabrication of a chemical product which is, or which comprises, the atomic system. If the decision is positive, the process is carried out in the real world, e.g. to fabricate the chemical product; that is, a real-world environment is created in which a physical transformation and/or chemical reaction occurs, which produces the chemical product.

Examples of this process have many applications. For example, calculated energy of molecules may be used to design a molecule or other chemical system (in silico), e.g. by screening a plurality of atomic systems based on an energy of the ground state of the structures to assess which atomic systems are likely to be relatively more stable than others.

Optionally a molecule or other chemical system may then be synthesized according to the atomic systems(s) identified as relatively more stable.

In another example, the energy calculation may be used to select a synthetic route for a molecule or other chemical system. For example there may be more than one possible synthetic route, and a ground state energy of one or more intermediates of each synthetic route may be determined and compared to assess which synthetic route is likely to be easier. Or there may be more than one possible synthetic route, and an energy of one or more different conformations of the same intermediate in each synthetic route may be determined and compared to assess which synthetic route is likely to be easier (the techniques described herein can be useful in determining accurate ground state energies for bent or twisted molecules). Optionally the molecule or other chemical system may then be synthesized using the easier synthetic route.

In another example, a synthetic route for a molecule or other chemical system may be known but the reaction mechanism may be unknown. One or more ground state energies may be determined for one or more components of one or more steps in a postulated mechanism, to evaluate a likelihood of the mechanism, and a mechanism for the reaction may be identified by comparing these. Once the reaction mechanism is known the reaction may be improved be adapting the mechanism; optionally a molecule or other chemical system may then be synthesized using the adapted mechanism.

In another example, a conformation of a molecule or other chemical system may be identified by comparing their energies for different postulated conformations. The molecule or other chemical system may then be modified to change the conformation or to make a desired conformation more likely; optionally the molecule or other chemical system may then be synthesized with the desired conformation.

In another example, the ground state energy may be determined for one or both of a ligand and its target. The target may be a bimolecular target and the ligand may be a candidate drug, or the ligand may be a catalyst. The ground state energies may be used to predict which ligands will interact strongly with the target; one or more of the ligands may then be synthesized for real-world evaluation.

In another example, the ground state energy may be determined for two or more different physical or electronic conformations of a molecule or other chemical system and a difference between the energies used to characterize the molecule/chemical system e.g. to identify an unknown molecule/chemical system or to design (and optionally then make) a molecule/chemical system with particular electromagnetic absorption or emission characteristics.

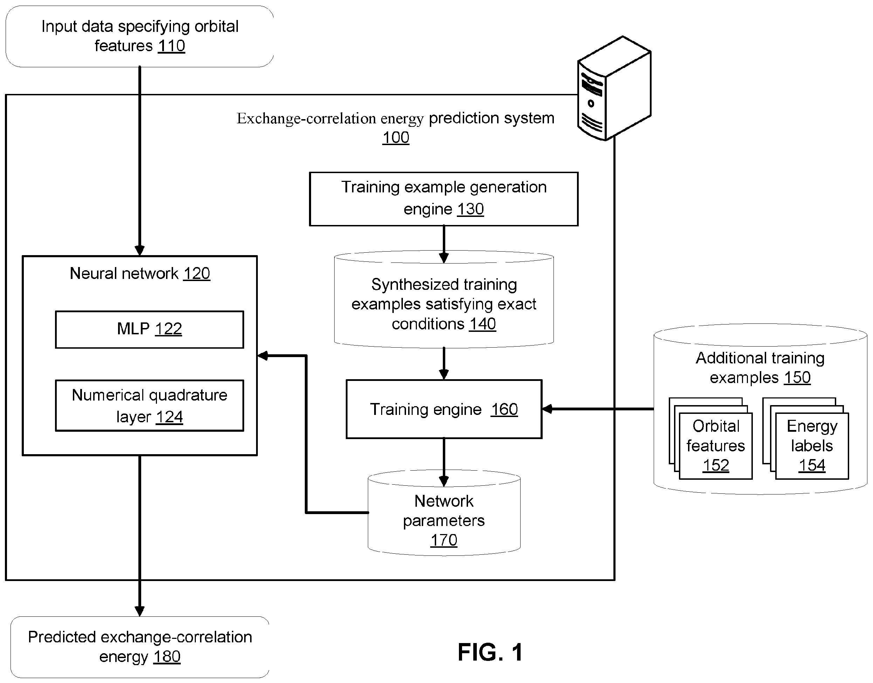

FIG. 1 shows an example of an exchange-correlation prediction system 100. The system 100 is an example of a system implemented as computer programs on one or more computers in one or more locations, in which the systems, components, and techniques described below can be implemented.

In general, the exchange-correlation prediction system 100 uses a neural network 120 to predict an exchange-correlation energy 180 of an atomic system based on input data 110 specifying the electron-orbital features of the atomic system. The network parameters 170 of the neural network 120 can be obtained by training the neural network 120 on a plurality of training examples. The atomic system is a system that includes one or more electrons in the presence of an external electric field. The electric field can be produced by one or more atomic nuclei, and the atomic system can represent a neutral or charged molecule or a neutral or charged single atom.

The electron-orbital features 110 of the atomic system include respective electron-orbital features of the atomic system at each of a plurality of grid points. The grid points can be grid points of any grid scheme suitable for three-dimensional numerical integration. In some implementations, the plurality of grid points include grid points on a real-space quadrature grid. Examples of specific grid schemes include the Treutler, Mura-Knowles, Delley, and Gauss-Chebyshev schemes.

In some implementations, the electron-orbital features 110 of a real-world atomic system can be obtained based on the molecular geometry of the atomic system (i.e. the 3D relative positions of the nuclei of the atomic system), which can be measured using techniques such as X-ray crystallography. In some implementations, one or more of the electron-orbital features 110 of the real-world atomic system can be experimentally measured. For example, orbital-dependent electron densities can be measured using techniques such as quantum crystallography or transmission electron microscopy. The phase symmetry of the electron density can be obtained by measuring the excitation distributions of electronic states in reciprocal space using angle-resolved photoelectron spectroscopy with circular polarized light, so that the full quantum mechanical wave function of the atomic system can be experimentally determined.

In some implementations, the electron-orbital features at the grid point position $\mathbf{r}$ can be features of a density matrix $\Gamma_{ab}^{\sigma}$ that is spin indexed $\sigma \in \{\uparrow, \downarrow\}$ and a basis set $\psi_a$ The basis set may be derived from data describing the molecular geometry of a plurality of molecules (that is, the relative (3D) positions of the nuclei of the molecules), including data which is determined by measurement, i.e. in real-world experiments conducted on at least some of the plurality of molecules. The method may include a preliminary step of receiving at least some of this measured data, and/or a preliminary step of obtaining at least some of it by measurement, followed by a step of deriving the basis set based on it. The features can include, for example, the density $\rho^{\sigma}(\mathbf{r}) = \Gamma_{ab}^{\sigma} \psi_a(\mathbf{r}) \psi_b(\mathbf{r})$ for each spin channel, the square norm of the gradient of the density in each spin channel and of the total density $|\nabla \rho^{\uparrow}|^2, |\nabla \rho^{\downarrow}|^2, |\nabla(\rho^{\uparrow} + \rho^{\downarrow})|^2$, the kinetic energy density $\tau^{\sigma}(\mathbf{r}) = \frac{1}{2} \Gamma_{ab}^{\sigma} [\nabla \psi_a(\mathbf{r}) \cdot \nabla \psi_b(\mathbf{r})]$ in each spin channel, and the local Hartree-Fock (HF) features

$$e_\sigma^{\omega HF}(\mathbf{r}) = -\frac{\Gamma_{ac}^\sigma \Gamma_{bd}^\sigma}{2} \int \psi_a(\mathbf{r})\psi_b(\mathbf{r}) \frac{\mathrm{erf}(\omega|\mathbf{r}-\mathbf{r}'|)}{|\mathbf{r}-\mathbf{r}'|} \psi_c(\mathbf{r}')\psi_d(\mathbf{r}')\mathrm{d}^3\mathbf{r}'$$ for each spin channel. The HF features can include range-separated (e.g., $\omega = 0.4$) HF features and non-range-separated ($\omega \to \infty$) HF features.

The system 100 can generate, for each of the plurality of grid points, a respective input feature vector for the electron-orbital features at the grid point, e.g., by concatenating multiple electron-orbital features. In a particular example, the system 100 can concatenate 11 electron-orbital features to form the input vector, as:

$$\mathbf{x}(\mathbf{r}) = \left(\rho^\uparrow, \rho^\downarrow, |\nabla\rho^\uparrow|^2, |\nabla\rho^\downarrow|^2, |\nabla(\rho^\uparrow+\rho^\downarrow)|^2, \tau^\uparrow, \tau^\downarrow, e_\uparrow^{HF}, e_\downarrow^{HF}, e_\uparrow^{\omega HF}, e_\downarrow^{\omega HF}\right)^T$$

In some implementations, the system 100 converts the electron-orbital features at the grid point from a linear scale to a logarithm scale by applying an element-wise squashing function on each input feature vector $\mathbf{x}(\mathbf{r})$. For example, the element-wise squashing function can take the form of $\log(|x_i| + \eta)$ where $\eta$ is a small constant (e.g., $10^{-4}$) to ensure numerical stability.

The system 100 processes the respective input feature vectors for the plurality of grid points using the neural network 120 to generate a predicted exchange-correlation energy of the atomic system.

The neural network 120 is trained on training data that includes a plurality of training examples. Each training example can correspond to a respective atomic system. In general, the training examples include a first subset of training examples that correspond to atomic systems having electron-orbital features and energy levels satisfying one or more mathematical constraint conditions.

In some implementations, the neural network 120 includes a multilayer perceptron (MLP) 122 and a numerical quadrature layer 124. The MLP 122 is configured to process each of the input feature vectors at the plurality of grid points to generate, for each input feature vector, a plurality of enhancement factors characterizing a plurality of contribution terms corresponding to the input feature vector.

In some implementations, the plurality of contribution terms include one or more of: a local-density approximation (LDA) exchange term, an HF term, or a range-separated HF term. The numerical quadrature layer 124 is configured to integrate the plurality of enhancement factors over the plurality of grid points to generate the predicted exchange-correlation energy 180 of the atomic system.

In some implementations, the neural network 120 further includes one or more layers in addition to the MLP 122 and the numerical quadrature layer 124.

In some implementations, the MLP includes an output linear layer that projects the input of the output linear layer to a vector of 3 elements followed by a scaled sigmoid layer that produces three enhancement factors f$_i$. In some implementations, the enhancement factors f$_i$ are bounded to fall within a specified range, e.g., a range of $0 < f_i < 2$. This range is inspired by the Lieb-Oxford bound and then empirically tuned.

In a particular example, after applying the element-wise squashing function on each input feature vector x(r), the system 100 passes the squashed input feature vector independently at each grid point through a linear layer and a *tanh* layer of the neural network 120 to produce an activation vector, and passes the activation vector through the MLP 122 to produce the enhancement factors f$_i$. The linear layer, the *tanh* layer, and the MLP are shared by the input feature vectors at the plurality of grid points.

In order to ensure the spin symmetry for the output of the neural network 122, in some implementations, the system 100 can process the input feature vectors with the same network twice - once for each ordering of the spin channels of the input features, and then averaging the two sets of the output enhancement factors. For example, an input feature vector with a first ordering of the spin channels can be

$$\mathbf{x}(\mathbf{r}) = \left(\rho^\uparrow, \rho^\downarrow, |\nabla\rho^\uparrow|^2, |\nabla\rho^\downarrow|^2, |\nabla(\rho^\uparrow+\rho^\downarrow)|^2, \tau^\uparrow, \tau^\downarrow, e_\uparrow^{HF}, e_\downarrow^{HF}, e_\uparrow^{\omega HF}, e_\downarrow^{\omega HF}\right)^T$$ and the input feature vector with a second ordering of spin channels can be

$$\mathbf{x}(\mathbf{r})' = \left(\rho^\downarrow, \rho^\uparrow, |\nabla\rho^\downarrow|^2, |\nabla\rho^\uparrow|^2, |\nabla(\rho^\uparrow+\rho^\downarrow)|^2, \tau^\downarrow, \tau^\uparrow, e_\downarrow^{HF}, e_\uparrow^{HF}, e_\downarrow^{\omega HF}, e_\uparrow^{\omega HF}\right)^T$$ The system 100 can

process each of $\mathbf{x}(\mathbf{r})$ and $\mathbf{x}(\mathbf{r})$' with the neural network 120 and average the two outputs to generate the predicted exchange-correlation energy. In some other implementations, the neural network 120 can be trained to become approximately spin symmetric in a single pass by randomly switching the order of spin channels in the input feature vector for each training example during training.

The numerical quadrature layer 124 is configured to integrate the plurality of enhancement factors over the plurality of grid points to generate the predicted exchange-correlation energy of the atomic system, as:

$$E_{xc}^{MLP}[\rho] = \int \mathbf{f}(\mathbf{x(r)}) \cdot \begin{bmatrix} e_x^{LDA}(\mathbf{r}) \\ e_x^{HF}(\mathbf{r}) \\ e_x^{\omega HF}(\mathbf{r}) \end{bmatrix} d^3\mathbf{r}, \qquad (2)$$

where $e_x^{LDA}(\mathbf{r})$, $e_x^{HF}(\mathbf{r})$, and $e_x^{\omega HF}(\mathbf{r})$ are contribution terms including the local-density approximation (LDA) exchange term, the HF term, and the range-separated HF term, respectively computed as:

$$e_x^{LDA}(\mathbf{r}) = -2\pi\left[(3/4\pi)\left(\rho^\uparrow + \rho^\downarrow\right)\right]^{4/3}, \ e_x^{HF}(\mathbf{r}) = e_{X,\uparrow}^{HF}(\mathbf{r}) + e_{X,\downarrow}^{HF}(\mathbf{r}) \qquad , \qquad \text{and}$$

$e_x^{\omega HF}(\mathbf{r}) = e_{X,\uparrow}^{\omega HF}(\mathbf{r}) + e_{X,\downarrow}^{\omega HF}(\mathbf{r})$. Here f(x(r)) denotes {$f_i$}, i.e. the output of the MLP 122 upon receiving the input feature vector x(r).

The integral form of the functional in Eq. (2) obeys the fundamental rotational and translational symmetries and size consistency required by the true functional.

In some implementations, since none of the input features are sensitive to long-range dispersion forces, the system 100 can augment the predicted energy with an empirical dispersion correction. In a particular implementation, the empirical dispersion correction is a Becke-Johnson damping with coefficients chosen to match those of B3LYP functional. The system 100 can add the correction as:

$$E_{xc}^{f} = E_{xc}^{MLP} + E_{D3(BJ)}. \qquad (3)$$

The system 100 or another system can include a training engine 160 to obtain the network parameters 170 of the neural network 120 based on a plurality of training examples. Each training example includes electron-orbital features and the corresponding ground-truth energy label of an atomic system.

In some implementations, one or more atomic systems in the training examples can be atomic systems with electron densities representing reactants and/or products for chemical reactions, including, for example, chemical reactions involving atomization, ionization, electron affinity, intermolecular binding energies of small main-group, and/or H-Kr molecules. The ground-truth energy label for an atomic system can be obtained based on reaction energy differences in a chemical reaction (r) involving the atomic system S. This reaction energy may be derived by measurement (i.e. in a real-world experiment carried out by studying the chemical reaction). The method may include a step of receiving the measured reaction energy, or a step of measuring it, and obtaining the ground-truth energy label from the measured reaction energy. For a given electron density $\rho_s$ of the atomic system $S$ and for the reaction $r$, the system 100 can generate the exchange-correlation energy label $\Delta E_{xc,r}^{*}$ by subtracting the computable parts of the energy in equation (1) from the reaction energy of the chemical reaction r.

In some implementations, the training examples can include one or more datasets expressing one or more exact constraints for the functional. That is, the plurality of training examples include a first subset of training examples that correspond to atomic systems that have electron-orbital features and energy levels satisfying one or more mathematical constraint conditions.

Certain mathematical constraint conditions, especially the exact conditions for systems with fractional electrons (such as the FC constraint condition and the FS constraint condition), have been shown to be challenging to address with manual design of the functionals. On the other hand, the mathematical constraint conditions can be illustrated as examples of virtual atomic systems that satisfy the mathematical constraint conditions. The system 100 can express the constraints as training data to train the functional to obey the constraints.

In some implementations, the system 100 or another training system includes a training example generation engine 130 to generate a set of synthesized training examples 140 by numerically synthesizing virtual atomic systems with electron-orbital features and energy levels satisfying the one or more mathematical constraint conditions.

In some implementations, the first subset of training examples can include an FC dataset with a set of training examples that satisfy the FC condition.

Based on the linearity of energy with a fractional electron number, the energy change in the reaction $X^{f+} \rightarrow (1 - f)X + fX^+$ is exactly zero for $0 \leq f \leq 1$ at a fixed atomic system X. The training example generation engine 130 can numerically synthesize training examples describing the above reactions for the FC dataset.

In general, the fractional electron conditions concern densities on the linear interpolation between the nearby integer ground states. To compute the kinetic energy density $\tau$ and the local HF features $e^{\omega HF}$, the training example generation engine 130 can convert the interpolated densities to Kohn-Sham orbitals. For example, to numerically synthesize a training example for the FC dataset, the training example generation engine 130 can use either a single neutral atom or (bounded) monatomic anion for H-A in the place of X and enumerate $f$ in steps of 0.01. Based on the linearity of the density, the training example generation engine 130 can synthesize the densities for $X^{f+}$ from a linear combination of densities for $X$ and $X^+$, and then use the Wu-Yang method to provide the Kohn-Sham orbitals for the fractional system with one fractional occupation for the frontier orbital, so that the kinetic energy density $\tau$ and the local HF features can be computed.

**In** some implementations, the first subset of training examples include an FS dataset with a set of training examples that satisfy the FS condition. The FS dataset can include training examples that describe a linear combination of the two degenerate spin states with total spin, $S$, and $m_S = \pm S$ , as

$$\rho[S, \gamma] = \left(\frac{1}{2} + \frac{\gamma}{2S}\right)\rho[S, S] + \left(\frac{1}{2} - \frac{\gamma}{2S}\right)\rho[S, -S]$$ with $\rho[S, m_s]$ denoting the electron density of each spin state and all states $-S \le \gamma \le S$ being degenerate in energy.

The training example generation engine 130 can use symmetry labels to ensure that the total occupation of each symmetry channel is the same for the two interpolation limits $p[S, \pm S]$. For example, in the case of the carbon triplet, the occupations $\rho[1,1]: [Be]2p_{x\uparrow}^1 2p_{y\uparrow}^1$ and $[1, -1]: [Be]2p_{x\downarrow}^1 2p_{y\downarrow}^1$ lead to the $\gamma$ = 0 occupation $\rho[1,0]: [Be]2p_{x\uparrow}^{1/2} 2p_{y\uparrow}^{1/2} 2p_{x\downarrow}^{1/2} 2p_{y\downarrow}^{1/2}$ . Note that this $\gamma$ = 0 state is seen in the limit of closed shell stretching of $C_2$, and the system can correctly label it with the same energy as the triplet.

To construct a dataset of zero-energy reactions $\rho[S, S] \to \rho[S, \gamma]$, the training example generation engine 130 can use all neutral atoms, cations and bounded anions for H-Ar with unpaired electrons. For each nucleus, the system enumerates $\gamma$ in steps of 0.01, and uses the Wu-Yang method to recover Kohn-Sham orbitals with fractional occupation from the interpolated density $\rho[S, \gamma]$.

In some implementations, the first subset of training examples can include a uniform electron gas (UEG) dataset with a set of training examples that satisfy the UEG condition. For example, to numerically synthesize the training examples in the UEG dataset, the training example generation engine 130 can apply the UEG constraint in the $(\rho^{\uparrow}, \rho^{\downarrow})$ plane from $(10^{-4}, 10^{-4})$ to $(10^{-1}, 10^{-1})$.

In a particular example, the UEG dataset includes training examples evenly spaced in a log space grid inside the lower half plane, which are augmented to cover the full plane by spin flipping. The UEG dataset can include additional training examples of fully polarized gas $10^{-4} \le \rho^{\uparrow} \le 10^{-1}$; $\rho^{\downarrow} = 0$( which are also augmented by spin flipping) and training examples of atomic systems located exactly on the un-polarized diagonal. The features for these systems can be computed as:

$$|\nabla\rho^{\sigma}| = 0$$

$$\tau^{\sigma}(\mathbf{r}) = \frac{3}{10}(3\pi^2)^{2/3}\rho^{\sigma}(\mathbf{r})^{5/3}$$

$$e_{x,\sigma}^{\omega HF} = -a_{\sigma}\left(\frac{6}{\pi}\right)^{1/3}\left[\sqrt{\pi}\mathrm{erf}(a_{\sigma}^{-1}) + a_{\sigma}(b_{\sigma} - c_{\sigma})\right]\rho^{\sigma}(\mathbf{r})^{4/3}$$

where $\alpha_{\sigma} = \omega/(6\pi^2\rho^{\sigma})^{1/3}$, $b_{\sigma} = \exp(-a_{\sigma}^{-2}) - 1$ and $c_{\sigma} = (a_{\sigma}^2 b_{\sigma} + 1)/2$.

In addition to the first subset of training examples that correspond to atomic systems satisfying the one or more mathematical constraint conditions, the training examples can further include a second subset of training examples 150 describing molecular properties of additional atomic systems.

The second subset of training examples 150 can include examples of molecules with electron-orbital features 152 and energy labels 154 obtained from literature and/or from computations. For example, for certain main-group molecules, the electron densities can be obtained from a popular traditional functional B3LYP, and the energy labels can be obtained from literature (e.g., benchmarked with real-world measurement data) or from computations with basis set CCSD(T) (coupled-cluster with single and double and perturbative triple excitations) calculations.

In some implementations, for an atomic system used for training, the system can compute the electron-orbital features on grid points of a plurality of different grid schemes, including, for example, the Treutler, Mura-Knowles, Delley, and Gauss-Chebyshev schemes. For the same atomic system, the features computed for the grid points based on each rid scheme can be used as an independent training example labeled with the same energy label, so that the trained neural network does not specialize to any particular choice of grid scheme.

The training engine 160 processes the electron-orbital features in the training example using the neural network 120 and in accordance with current values of the network parameters to generate a predicted exchange-correlation energy for the training example.

The training engine 160 further computes a gradient of a training loss with respect to the network parameters. The training loss includes a regression loss that measures, for each of one or more of the training examples, an error between the predicted exchange-correlation energy for the training example and the ground-truth energy label derived from the reaction energy in the training example.

In some implementations, the training loss further includes a self-consistent field (SCF) loss that represents a computed energy of an atomic system subject to electron number conservation.

A common concern with deep learned functionals is that the functional derivatives may be noisy, prohibiting use in self-consistent optimization. The training engine 160 can address this challenge by regularizing the functional derivative.

In some implementations, the overall training loss can be a weighted sum of the regression loss and the SCF loss, as:

$$\mathcal{L} = \mathbb{E}\left[\left(\Delta E^f_{\text{xc},r} - \Delta E^*_{\text{xc},r}\right)^2\right] + \lambda\mathbb{E}\left[\delta E^2_{\text{SCF},s}\right] \qquad (4)$$

where $\mathbb{E}$ denotes the expectation over the dataset of reactions ($r$) or systems ($s$), and the hyperparameter $\lambda$ controls the weighting of the gradient term.

For the regression loss, $\Delta E^{\text{f}}_{\text{xc},r}$ denotes the model's prediction for the total reaction exchange-correlation energy (product minus reactant exchange-correlation energy) and $\Delta E^*_{\text{xc},r}$ denotes the ground-truth exchange-correlation energy for the reaction.

In some implementations, to improve computation efficiency, the training engine 160 can compute the SCF loss using a second-order perturbation theory approach. Overall, the computation process includes constructing the Roothan-Hall equations using a Fock matrix F constructed from the neural network's derivatives, and stipulating that the solution is close to reasonable Kohn-Sham orbitals from a traditional functional.

More specifically, the training engine 160 can use the Roothan-Hall equations to derive an approximate expression for the change in energy after a single SCF iteration starting from orbitals from a traditional functional (e.g., the B3LYP functional). The term $\delta E_{\text{SCF}}$ in equation (3) can be computed as:

$$\delta E_{\text{SCF}} = \frac{1}{2}\sum_{i\neq j}\frac{n_i - n_j}{\epsilon_i - \epsilon_j}[\mathbf{C}^\top\mathbf{F}\mathbf{C}]^2_{ij}, \qquad (5)$$

where $n$, $\epsilon$ are the orbital occupations and energies, and C is a reasonable guess for the orbital coefficients taken from a traditional functional (the spin index is dropped for clarity).

In some implementations, the training engine 160 computes the SCF loss based on an SCF dataset that includes atomic systems for which the regularization term in equation (5) can be computed efficiently.

That is, the plurality of training examples further include a third subset of training examples for computing the SCF loss. To evaluate the Fock matrix $F^\sigma_{ab} = \left(\frac{dE}{dx}\right) \cdot \left(\partial\mathbf{x}/\partial\Gamma^\sigma_{ab}\right)$ during training, the training engine 160 can precompute and store the input feature derivatives $\left(\partial\mathbf{x}/\partial\Gamma^\sigma_{ab}\right)$. The derivatives for the local HF $e^{\omega\text{HF}}$ features can be obtained based on:

$$\chi^{\sigma,\omega}_a(\mathbf{r}) = \Gamma^\sigma_{bd}\psi_b(\mathbf{r})\int\frac{\text{erf}(\omega|\mathbf{r}-\mathbf{r}'|)}{|\mathbf{r}-\mathbf{r}'|}\psi_a(\mathbf{r}')\psi_d(\mathbf{r}')\text{d}^3\mathbf{r}', \qquad (6)$$

such that the full gradient $\partial e^{\omega\text{HF}}_\sigma/\partial\Gamma^{\sigma'}_{ab} = -\psi_a\chi^{\omega,\sigma}_b\delta_{\sigma\sigma'}$ can be computed on the fly.

The SCF dataset can include training examples describing atomic systems that are included in the regression training examples (i.e., the training examples used for computing the regression loss). For example, for atoms and diatomic molecules in the regression training examples, the system can generate the features for the SCF loss at PySCF grid level 2 and use the largest basis set in the aug-pc-X family such that the number of basis functions is less than 128. For larger neutral molecules in the regression training examples, the training engine 160 can use grid level 1 and the largest basis set with less than 128 basis functions from cc-pCV(Q+d)Z, *ccpCV(T + d)Z, cc - pV(T + d)Z* or cc-pV(D+d)Z. In some implementations, the training engine 160 excludes any atomic systems with a large number of grid points (e.g., > 32,000 grid points) for the SCF loss computation.

The training engine 160 updates the current values of the network parameters 170 using the gradient of the training loss. The training engine 160 can update the parameters through any appropriate backpropagation-based machine learning technique, e.g., using the Adam or AdaGrad optimizers.

In some implementations, the training engine 160 can repeatedly perform training on different batches of training examples sampled from the training datasets to repeatedly update the values of the parameters of the neural network. Each training batch can include a set of randomly selected SCF training examples (selected from the third subset of training examples) and a set of randomly selected regression training examples (selected from the first and second subset of training examples), so that the training engine 160 can compute both the regression loss and the SCF loss on the training batch.

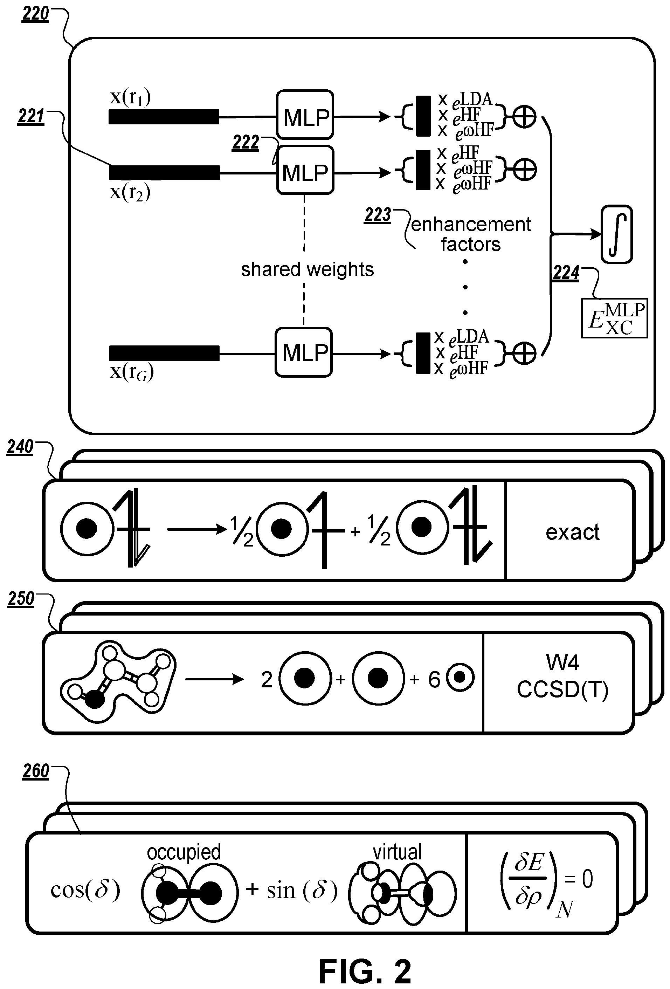

FIG. 2 illustrates examples of processes of predicting an exchange-correlation energy of an atomic system and training a neural network for predicting the exchange-correlation energy. For convenience, the processes illustrated in FIG. 2 will be described as being performed by a system of one or more computers located in one or more locations. For example, an exchange-correlation prediction system, e.g., the exchange-correlation prediction system 100 of FIG. 1, appropriately programmed in accordance with this specification, can perform these processes.

As shown in FIG. 2, in the prediction process 220, the system uses a neural network including an MLP 222 to process the input feature vectors 221 at a plurality of grid points to generate enhancement factors 223 for contribution terms for each respective grid point. The system integrates a weighted sum of contribution terms scaled by the enhancement factors over the plurality of grid points to generate the predicted exchange-correlation energy 224 of the atomic system.

The system can train the neural network using training examples including a first subset of training examples 240, a second subset of training examples 250, and a third subset of training examples 260. The first subset of training examples 240 include training examples that correspond to atomic systems having electron-orbital features and energy levels satisfying one or more mathematical constraint conditions. The second subset of training examples 250 include additional training examples of atomic systems with electron-orbital features and energy labels obtained from literature (e.g., benchmarked by real-world measurement data) and from computation (e.g., based on CCSD calculations). The third subset of training examples 260 include training examples for computing the SCF loss and include atomic systems for which the regularization term in equation (5) can be computed efficiently.

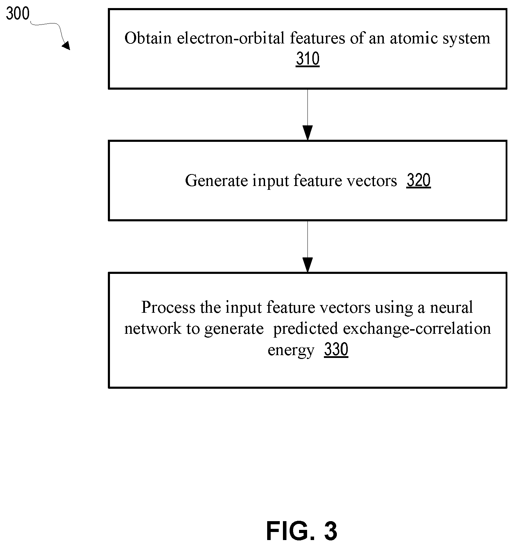

FIG. 3 is a flow diagram illustrating an example process 300 for predicting an exchange-correlation energy of an atomic system. For convenience, the process 300 will be described as being performed by a system of one or more computers located in one or more locations. For example, an exchange-correlation prediction system, e.g., the exchange-correlation prediction system 100 of FIG. 1, appropriately programmed in accordance with this specification, can perform the process 300.

In step 310, the system obtains electron-orbital features of an atomic system. In particular, the system obtains respective electron-orbital features of the atomic system at each of a plurality of grid points.

The grid points can be grid points of any grid scheme suitable for three-dimensional numerical integration. In some implementations, the plurality of grid points include grid points on a real-space quadrature grid. Examples of specific grid schemes include the Treutler, Mura-Knowles, Delley, and Gauss-Chebyshev schemes.

In some implementations, the electron-orbital features obtained for the atomic system include one or more of: an electron density distribution, an electron density gradient norm distribution, a kinetic energy density distribution, a local Hartree-Fock (HF) exchange distribution, or a range-separated form of the local HF exchange distribution of the atomic system.

In step 320, the system generates, for each of the plurality of grid points, a respective input feature vector for the electron-orbital features at the grid point.

The system can concatenate multiple electron-orbital features for the atomic system at the grid point to form the input feature vector for the grid point. In some implementations, the system converts the electron-orbital features at the grid point from a linear scale to a logarithm scale by applying an element-wise squashing function on each input feature vector.

In step 330, the system processes the respective input feature vectors for the plurality of grid points using a neural network to generate a predicted exchange-correlation energy of the atomic system.

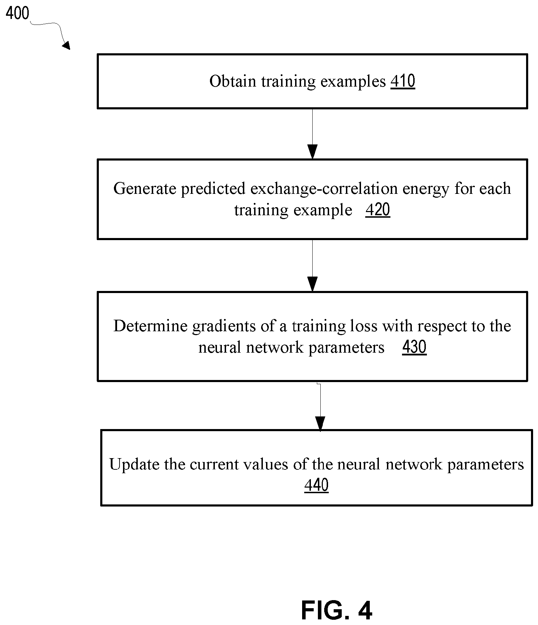

In some implementations, the neural network is trained on training data that includes a plurality of training examples. Each training corresponds to a respective atomic system. Examples of the training process are described with reference to FIG. 4. In general, the training examples include a first subset of training examples that correspond to atomic systems having electron-orbital features and energy levels satisfying one or more mathematical constraint conditions.

In some implementations, the neural network includes a multilayer perceptron (MLP) and a numerical quadrature layer. The MLP is configured to process each of the input feature vectors at the plurality of grid points to generate a plurality of enhancement factors characterizing a plurality of contribution terms corresponding to the input feature vector. The numerical quadrature layer is configured to integrate a weighted sum of the plurality of contribution terms scaled by the corresponding enhancement factors over the plurality of grid points to generate the predicted exchange-correlation energy of the atomic system.

In some implementations, the plurality of contribution terms include one or more of: a local-density approximation (LDA) exchange term, an HF term, or a range-separated HF term.

In some implementations, the system augments the predicted exchange-correlation energy with an empirical dispersion correction.

FIG. 4 is a flow diagram illustrating an example process 400 for training a neural network for predicting exchange-correlation energy of an atomic system. For convenience, the process 400 will be described as being performed by a system of one or more computers located in one or more locations. For example, the training engine 160 of FIG. 1, appropriately programmed in accordance with this specification, can perform the process 400.

Referring to FIG. 4, in step 410, the training engine obtains a plurality of training examples. Each training example includes electron-orbital features and the corresponding ground-truth energy label of an atomic system.

In some implementations, the training examples can include one or more datasets expressing one or more exact constraints for the functional. That is, the plurality of training examples include a first subset of training examples that correspond to atomic systems that have electron-orbital features and energy levels satisfying one or more mathematical constraint conditions.

In general, the training engine can include any combinations of one or more specific constraint conditions for the first subset of training examples to suit a particular range of applications. For example, in some implementations, the one or more mathematical constraint conditions include: a uniform electron gas (UEG) constraint condition, a fractional charge (FC) constraint condition, or a fractional spin (FS) constraint condition.

In some implementations, the one or more mathematical constraint conditions include both the FC constraint condition and the FS constraint condition.

In some implementations, the training engine generates the first subset of training examples by numerically synthesizing virtual atomic systems with electron-orbital features and energy levels satisfying the one or more mathematical constraint conditions.

In addition to the first subset of training examples that correspond to atomic systems satisfying the one or more mathematical constraint conditions, the training examples can further include a second subset of training examples describing molecular properties of additional atomic systems.

The second subset of training examples can include examples of molecules with electron-orbital features and energy labels obtained from literature (e.g. reporting experiments on the molecules in the real world, which made measurements on the real-world molecules of data from which the electron-orbital features and energy labels are derived; in a variant, the method may include a preliminary step of receiving these measurements, or of making these measurements, and from the measured data deriving the electron-orbital features and energy labels) and from computation. For example, for certain main-group molecules, the electron densities can be obtained from a popular traditional functional B3LYP, and the energy labels can be obtained from literature (e.g., benchmarked with real-world measurement data) or based on computation basis set CCSD(T) (coupled-cluster with single and double and perturbative triple excitations) calculations).

In some implementations, for an atomic system used for training, the training engine can compute the electron-orbital features on grid points of a plurality of different grid schemes, including, for example, the Treutler, Mura-Knowles, Delley, and Gauss-Chebyshev schemes. For the same atomic system, the features computed for the grid points based on each rid scheme can be used as an independent training example labeled with the same reaction energy, so that the model does not specialize to any particular choice of grid scheme.

In step 420, for each training example, the training engine processes the electron-orbital features in the training example using the neural network and in accordance with current values of the parameters to generate a predicted exchange-correlation energy for the training example.

In step 430, the training engine computes a gradient of a training loss with respect to the parameters. The training loss includes a regression loss that measures, for each of one or more of the training examples, an error between the predicted exchange-correlation energy for the training example and the ground-truth energy label derived from the reaction energy in the training example.

In some implementations, the training loss further includes a self-consistent field (SCF) loss that represents a computed energy of an atomic system subject to electron number conservation.

In some implementations, to improve computation efficiency, the training engine can compute the SCF loss using a second-order perturbation theory approach.

In some implementations, the training engine computes the SCF loss based on an SCF dataset that includes atomic systems which the regularization term in equation (4) can be computed efficiently. That is, the plurality of training examples further include a third subset of training examples for computing the SCF loss.

In step 440, the system updates the current values of the parameters using the gradient. The system can update the parameters through any appropriate backpropagation-based machine learning technique, e.g., using the Adam or AdaGrad optimizers.

The training engine can repeatedly perform steps 420-440 on different batches of training examples sampled from the training datasets to repeatedly update the values of the parameters of the neural network. For example, the training engine can continue to perform the steps 420-440 until a threshold number of iterations have been performed, until the parameter values have converged, or until some other termination criterion is satisfied. In some implementations, each training batch can include a set randomly selected SCF training examples (selected from the third subset of training examples) and a set of randomly selected regression training examples (selected from the first and second subset of training examples), so that the training engine can compute both the regression loss and the SCF loss on the training batch.

Further performances of example implementations of the described techniques for predicting properties of atomic systems is illustrated. In particular, the performance of 4 trained variants of an exchange-correlation energy prediction neural network (e.g, the NN 120 shown in FIG. 1) for predicting the exchange-correlation energy of atomic systems based on the respective electron-orbital features of the atomic systems is demonstrated. The functionals corresponding to these trained variants of the NN are respectively labeled as DM20m, DM20mu, DM20mc, and DM20mcs. The difference between these functionals is in the choice of which exact constraints to include in the training data. The suffix m denotes the inclusion of main group molecule reactions. The suffixes u, c, and s indicate inclusion of UEG, FC and FS constraints, respectively. Overall, these 4 trained functionals are termed DM20 functionals.

The performances of the DM20 functionals are evaluated and compared to other known functionals on several benchmark dataset. One of the benchmark datasets is the GMTKN55 database for general main group thermochemistry, as described in Goerigk, L. et al. "A look at the density functional theory zoo with the advanced GMTKN55 database for general main group thermochemistry, kinetics and noncovalent interactions". Phys. Chem. Chem. Phys. 19,32184-32215 (2017). The reactions of this dataset are categorized into 5 classes, including: reaction energies for small systems, large systems and isomerization reactions, reaction barrier heights, intermolecular non covalent interaction (NCI), and intramolecular NCI.

Performance metrics of the DM20 functionals compared with several other functionals computed on benchmark data is of interest. The mean of means (MoM) error metric of the DM20 functionals compared to the best performing functionals at Rungs 2-5 from Goerigk, L. et al. for each class of reactions of the GMTKN55 benchmark dataset is of particular interest. These result demonstrate that the DM20 functionals exhibit state-of-the-art performance on GMTKN55, with improvement over the best hybrid functional (PW6B95:D3$_0$ ) in all 5 classes. Inclusion of the UEG constraint in DM20mu provides a slightly better performance on MoM, but overall DM20m and DM20mu show similar behavior on GMTKN55. Both demonstrate extremely good performance on the "large" subclass, which is completely absent from the training set, providing strong evidence of generalization beyond the training data.

The performance metrics (including MoM, WTMAD-1, and WTMAD-2) of the DM20 functionals compared with 4 high-performing functionals is of interest. The performances are evaluated on 3 benchmark datasets including the GMTKN55 dataset, a dimer bond breaking dataset, and the QM9 dataset. The data revealed that the traditional functionals produce large prediction errors for atomic systems with mobile charge or spin. The DM20 functionals demonstrate superior performances for the atomic systems for which the traditional functionals fail.

The comparison of performance metrics of the functionals for the large QM9 dataset is of interest, which includes a complete enumeration of all closed shell neutral molecules containing {H, C, N, O,, F }, that can be constructed using up to 9 heavy atoms. Results in table 5b demonstrate that all DM20 functionals have errors less than 1.7kcal/mol, considerably lower than the best conventional functional $\omega$B97X (2.1kcal/mol). The strong performance on QM9 demonstrate the generalization of the DM20 functionals to disparate distributions of molecules.

Certain aspects of performances of the DM20 functionals compared with several other functionals is of interest together with data related to the FC constraint and charge delocalization errors, also withdata related to the FS constraint and restricted bond breaking. The DM20mc functional correctly captures the piecewise linear energy of a H atom as the electron number is varied from neutral by $\Delta N_{elec.}$ , i.e. revealing the deviation from linear behavior for simple atoms (H, C) and small molecules together with the correct handling of the fractionally charged states generalizes to improved cation binding curves for DM20mc(s), whereinthe B3LYP functional produces a spurious delocalization of the hole for both propane and ethane dimer cation, because the fractional charge error is larger than the difference in ionization potentials. By contrast, the DM20mc functional correctly removes almost all the charge from the longer alkane, i.e the individual binding curves for the single bond of $H_2$ and triple bond of $N_2$, and demonstrate the improved performance of the DM20mcs functional on restricted binding curves, i.e. revealinga binding curve for the H_50 chain of atoms with the def2TZVP basis set, and demonstrates the generalization of DM20mcs to larger systems.

Several other aspects of performances of the DM20 functionals is of interest, i.e.data related to the conrotatory and disrotatory pathways of bicyclobutane isomerisation. The HOMO of a single spin channel in an unrestricted calculation is shown for the transition states. Spin is delocalized across two atoms in the conrotatory path, requiring the FS constraint being met for accurate modelling. Panel 7b demonstrate the importance of the UEG condition for selected ring and cage systems. Isosurfaces at a density difference of 0.001 (atomic units) show that DM20mu puts more density in low gradient (low hessian) regions that occur at ring and cage centers. The effect is more pronounced in strained structures and correlates with the atomization energy error benchmarked with G4(MP2) labels, revealingthat exact constraints improve performance on challenging chemistry.

By this, the state-of-the-art performances of the described techniques compared with conventional functionals for predicting energies of atomic systems is illustrated. The performances metrics benchmarked on several large datasets show that the described techniques allow behavior learned from training on fictional atomic systems to generalize to larger real molecules. The constraints are demonstrated to contribute to superior approximations of the exchange-correlation energies of a broad range of atomic systems.

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions. Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory storage medium for execution by, or to control the operation of, data processing apparatus. The computer storage medium can be a machine-readable storage device, a machine-readable storage substrate, a random or serial access memory device, or a combination of one or more of them. Alternatively or in addition, the program instructions can be encoded on an artificially generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a data communication network.

In this specification, the term "database" is used broadly to refer to any collection of data: the data does not need to be structured in any particular way, or structured at all, and it can be stored on storage devices in one or more locations. Thus, for example, the index database can include multiple collections of data, each of which may be organized and accessed differently.

Similarly, in this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Data processing apparatus for implementing machine learning models can also include, for example, special-purpose hardware accelerator units for processing common and compute-intensive parts of machine learning training or production, i.e., inference, workloads.

Machine learning models can be implemented and deployed using a machine learning framework, .e.g., a TensorFlow framework.

Embodiments of the subject matter described in this specification can be implemented in a computing system that includes a back end component, e.g., as a data server, or that includes a middleware component, e.g., an application server, or that includes a front end component, e.g., a client computer having a graphical user interface, a web browser, or an app through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

Particular embodiments of the subject matter have been described. The invention is defined by the appended claims.

**Claims**

1. A method performed by one or more computers and for predicting an exchange-correlation energy (180, 224) of an atomic system, the method comprising:

   obtaining respective electron-orbital features (110) of the atomic system at each of a plurality of grid points;
   generating, for each of the plurality of grid points, a respective input feature vector (221) for the electron-orbital features (110) at the grid point;
   processing the respective input feature vectors (221) for the plurality of grid points using a neural network (120) to generate a predicted exchange-correlation energy (180, 224) of the atomic system; and
   wherein the neural network (120) is trained on training data that includes a plurality of training examples (140, 150, 240, 250, 260), the training examples (140, 150, 240, 250, 260) each corresponding to a respective atomic system, and the training examples (140, 150, 240, 250, 260) including a first subset of training examples (140, 240) that correspond to atomic systems that have electron-orbital features and energy levels satisfying one or more mathematical constraint conditions, the one or more mathematical constraint conditions including: a uniform electron gas (UEG) constraint condition, a fractional charge (FC) constraint condition, or a fractional spin (FS) constraint condition;
   and either:

      one or more of the electron-orbital features (110, 152) of the atomic system are obtained based on real-world measurement data; or

the method further comprises determining based on the predicted exchange-correlation energy (180, 224) of the atomic system, whether to perform a fabrication process of a chemical product which comprises the atomic system, and, if the determination is positive, causing the fabrication to be performed.

2.  The method of claim1, wherein:
    the atomic systems corresponding to the plurality of training examples (140, 150, 240, 250, 260) include synthetically generated virtual atomic systems with electron-orbital features and energy levels satisfying the one or more mathematical constraint conditions.

3.  The method of any one of claims 1 or 2 wherein the training examples (140, 150, 240, 250, 260) are associated with data describing physical properties of the corresponding atomic system, the data for a plurality of the training examples (150, 250) having been obtained by measurements performed in the real world on the corresponding atomic systems.

4.  The method of any one of claims 1-3, wherein:
    the plurality of grid points include grid points on a real-space quadrature grid.

5.  The method of any one of claims 1-4, wherein:
    the electron-orbital features (110) obtained for the atomic system include one or more of: an electron density distribution, an electron density gradient norm distribution, a kinetic energy density distribution, a local Hartree-Fock (HF) exchange distribution, or a range-separated form of the local HF exchange distribution of the atomic system.

6.  The method of any one of claims 1-5, wherein generating the input feature vector (221) for each of the plurality of grid points includes:

    converting the electron-orbital features (110) at the grid point from a linear scale to a logarithm scale; and concatenating the electron-orbital features (110) in the logarithm scale to form the input feature vector (221) for the grid point.

7.  The method of any one of claims 1-6, wherein the neural network (120) includes:

    a multilayer perceptron (MLP) (122, 222) configured to process each of the input feature vectors (221) at the plurality of grid points to generate a plurality of enhancement factors (223) characterizing a plurality of contribution terms corresponding to the input feature vector (221); and
    a numerical quadrature layer (124) configured to integrate a weighted sum of the plurality of contribution terms scaled by the enhancement factors (223) over the plurality of grid points to generate the predicted exchange-correlation energy (180, 224) of the atomic system.

8.  The method of claim 7, wherein:
    the plurality of contribution terms include one or more of: a local-density approximation (LDA) exchange term, an HF term, or a range-separated HF term.

9.  A computer-implemented method for training the neural network (120) of any one of claims 1-8, the neural network (120) having a plurality of parameters (170), and the method comprising:

    obtaining a plurality of training examples (140, 150, 240, 250, 260), each training example (140, 150, 240, 250, 260) including electron-orbital features (152) and corresponding ground-truth energy label (154) of an atomic system, the plurality of training examples (140, 150, 240, 250, 260) including a first subset of training examples (140, 240) that correspond to atomic systems that have electron-orbital features and energy levels satisfying one or more mathematical constraint conditions, wherein the one or more mathematical constraint conditions include: a uniform electron gas (UEG) constraint condition, a fractional charge (FC) constraint condition, or a fractional spin (FS) constraint condition;
    for each training example (140, 150, 240, 250, 260), processing the electron-orbital features in the training example (140, 150, 240, 250, 260) using the neural network (120) and in accordance with current values of the parameters (170) to generate a predicted exchange-correlation energy (180, 224) for the training example (140, 150, 240, 250, 260);
    determining a gradient with respect to the parameters (170) of a training loss, the training loss including a regression loss that measures, for each training example (140, 150, 240, 250, 260), an error between the predicted exchange-correlation energy (180, 224) for the training example and the ground-truth energy label (154) in the training example; and

updating the current values of the parameters (170) using the gradient.

10. The method of claim 9, wherein:
the one or more mathematical constraint conditions include a fractional charge (FC) constraint condition and a fractional spin (FS) constraint condition.

11. The method of claim 9 or 10, wherein:
the training loss further includes a self-consistent field (SCF) loss, the SCF loss representing a calculated energy of the atomic system subject to electron number conservation.

12. The method of any one of claims 9-11, further comprising:
generating the first subset of training examples (140, 240) by numerically synthesizing virtual atomic systems with electron-orbital features and energy levels satisfying the one or more mathematical constraint conditions.

13. The method of any preceding claim, in which the electron-orbital features (110, 152) at each grid point are features of a density matrix $\Gamma_{ab}^{\sigma}$ that is spin indexed $\sigma \in \{\uparrow, \downarrow\}$ and based on a basis set $\psi_a$, the basis set having been derived from data describing a molecular geometry of a plurality of molecules, including data which is determined by real-world measurements.

14. A system (100) comprising:

one or more computers; and
one or more storage devices storing instructions that when executed by the one or more computers, cause the one or more computers to perform the operations of the respective method of any one of claims 1-13.

15. One or more computer-readable storage media storing instructions that, when executed by one or more computers, cause the one or more computers to perform the operations of the respective method of any one of claims 1-13.

**Patentansprüche**

1. Verfahren, das durch einen oder mehrere Computer durchgeführt wird und zum Vorhersagen einer Austausch-Korrelationsenergie (180, 224) eines Atomsystems dient, wobei das Verfahren Folgendes umfasst:

Erlangen jeweiliger elektronen-orbitaler Merkmale (110) des Atomsystems an jedem einer Vielzahl von Gitterpunkten;
Erzeugen eines jeweiligen Eingabe-Merkmalsvektors (221) für die elektronen-orbitalen Merkmale (110) an dem Gitterpunkt für jeden der Vielzahl von Gitterpunkten;
Verarbeiten der jeweiligen Eingabe-Merkmalsvektoren (221) für die Vielzahl von Gitterpunkten unter Verwendung eines neuronalen Netzwerks (120), um eine vorhergesagte Austausch-Korrelationsenergie (180, 224) des Atomsystems zu erzeugen; und
wobei das neuronale Netzwerk (120) auf Trainingsdaten trainiert wird, die eine Vielzahl von Trainingsbeispielen (140, 150, 240, 250, 260) beinhalten, wobei die Trainingsbeispiele (140, 150, 240, 250, 260) jeweils einem jeweiligen Atomsystem entsprechen und die Trainingsbeispiele (140, 150, 240, 250, 260) eine erste Teilmenge von Trainingsbeispielen (140, 240) beinhalten, die Atomsystemen entsprechen, die elektronen-orbitale Merkmale und Energiestufen aufweisen, die eine oder mehrere mathematische Zwangsbedingungen erfüllen, wobei die eine oder die mehreren mathematischen Zwangsbedingungen Folgendes beinhalten: eine Zwangsbedingung für ein einheitliches Elektronengas (uniform electron gas, UEG), eine Zwangsbedingung für eine fraktionale Ladung (fractional charge, FC) oder eine Zwangsbedingung für einen fraktionalen Spin (fractional spin, FS); und entweder:

ein oder mehrere der elektronen-orbitalen Merkmale (110, 152) des Atomsystems basierend auf Messdaten der realen Welt erlangt werden; oder
das Verfahren ferner das Bestimmen basierend auf der vorhergesagten Austausch-Korrelationsenergie (180, 224) des Atomsystems, ob ein Herstellungsprozess eines chemischen Produkts, das das Atomsystem umfasst, durchgeführt werden soll, und, falls die Bestimmung positiv ist, das Veranlassen, dass die Herstellung durchgeführt wird, umfasst.

2. Verfahren nach Anspruch 1, wobei:

die Atomsysteme, die der Vielzahl von Trainingsbeispielen (140, 150, 240, 250, 260) entsprechen, synthetisch erzeugte virtuelle Atomsysteme mit elektronen-orbitalen Merkmalen und Energiestufen beinhalten, die die eine oder die mehreren mathematischen Zwangsbedingungen erfüllen.

3. Verfahren nach einem der Ansprüche 1 oder 2, wobei die Trainingsbeispiele (140, 150, 240, 250, 260) Daten zugeordnet sind, die physikalische Eigenschaften des entsprechenden Atomsystems beschreiben, wobei die Daten für eine Vielzahl der Trainingsbeispiele (150, 250) durch Messungen erlangt worden sind, die in der realen Welt an den entsprechenden Atomsystemen durchgeführt wurden.

4. Verfahren nach einem der Ansprüche 1 bis 3, wobei:

die Vielzahl von Gitterpunkten Gitterpunkte auf einem Quadraturgitter im realen Raum beinhaltet.

5. Verfahren nach einem der Ansprüche 1 bis 4, wobei:

die für das Atomsystem erlangten elektronen-orbitalen Merkmale (110) eines oder mehrere von Folgendem beinhalten: eine Elektronendichteverteilung, eine Elektronendichtegradienten-Normverteilung, eine kinetische Energiedichteverteilung, eine lokale Hartree-Fock- (HF-) Austauschverteilung oder eine bereichsgetrennte Form der lokalen HF-Austauschverteilung des Atomsystems.

6. Verfahren nach einem der Ansprüche 1 bis 5, wobei das Erzeugen des Eingabe-Merkmalsvektors (221) für jeden der Vielzahl von Gitterpunkten Folgendes beinhaltet:

Umwandeln der elektronen-orbitalen Merkmale (110) an dem Gitterpunkt von einer linearen Skala in eine logarithmische Skala; und

Verketten der elektronen-orbitalen Merkmale (110) in der logarithmischen Skala, um den Eingabe-Merkmalsvektor (221) für den Gitterpunkt zu bilden.

7. Verfahren nach einem der Ansprüche 1 bis 6, wobei das neuronale Netzwerk (120) Folgendes beinhaltet:

ein mehrschichtiges Perzeptron (multilayer perceptron, MLP) (122, 222), das dazu konfiguriert ist, jeden der Eingabe-Merkmalsvektoren (221) an der Vielzahl von Gitterpunkten zu verarbeiten, um eine Vielzahl von Verstärkungsfaktoren (223) zu erzeugen, die eine Vielzahl von Beitragsausdrücken entsprechend dem Eingabe-Merkmalsvektor (221) kennzeichnen; und

eine numerische Quadraturschicht (124), die dazu konfiguriert ist, eine gewichtete Summe der Vielzahl von mit den Verstärkungsfaktoren (223) skalierten Beitragsausdrücken über die Vielzahl von Gitterpunkten zu integrieren, um die vorhergesagte Austausch-Korrelationsenergie (180, 224) des Atomsystems zu erzeugen.

8. Verfahren nach Anspruch 7, wobei:

die Vielzahl von Beitragsausdrücken eines oder mehrere von Folgendem beinhaltet: einen Local-Density-Approximation- (LDA-) Austauschausdruck, einen HF-Ausdruck oder einen bereichsgetrennten HF-Ausdruck.

9. Computerimplementiertes Verfahren zum Trainieren des neuronalen Netzwerks (120) nach einem der Ansprüche 1 bis 8, wobei das neuronale Netzwerk (120) eine Vielzahl von Parametern (170) aufweist und das Verfahren Folgendes umfasst:

Erlangen einer Vielzahl von Trainingsbeispielen (140, 150, 240, 250, 260), wobei jedes Trainingsbeispiel (140, 150, 240, 250, 260) elektronen-orbitale Merkmale (152) und ein entsprechendes Grundwahrheits-Energieetikett (154) eines Atomsystems beinhaltet, wobei die Vielzahl von Trainingsbeispielen (140, 150, 240, 250, 260) eine erste Teilmenge von Trainingsbeispielen (140, 240) beinhaltet, die Atomsystemen entsprechen, die elektronen-orbitale Merkmale und Energiestufen aufweisen, die eine oder mehrere mathematische Zwangsbedingungen erfüllen, wobei die eine oder die mehreren mathematischen Zwangsbedingungen Folgendes beinhalten: eine Zwangsbedingung für ein einheitliches Elektronengas (UEG), eine Zwangsbedingung für eine fraktionale Ladung (FC) oder eine Zwangsbedingung für einen fraktionalen Spin (FS);

für jedes Trainingsbeispiel (140, 150, 240, 250, 260) das Verarbeiten der elektronen-orbitalen Merkmale in dem Trainingsbeispiel (140, 150, 240, 250, 260) unter Verwendung des neuronalen Netzwerks (120) und gemäß den aktuellen Werten der Parameter (170), um eine vorhergesagte Austausch-Korrelationsenergie (180, 224) für das Trainingsbeispiel (140, 150, 240, 250, 260) zu erzeugen;

Bestimmen eines Gradienten in Bezug auf die Parameter (170) eines Trainingsverlustes, wobei der Trainingsverlust einen Regressionsverlust beinhaltet, der für jedes Trainingsbeispiel (140, 150, 240, 250, 260) einen Fehler zwischen der vorhergesagten Austausch-Korrelationsenergie (180, 224) für das Trainingsbeispiel und dem Grundwahrheits-Energieetikett (154) in dem Trainingsbeispiel misst; und

Aktualisieren der aktuellen Werte der Parameter (170) unter Verwendung des Gradienten.

10. Verfahren nach Anspruch 9, wobei:
die eine oder die mehreren mathematischen Zwangsbedingungen eine Zwangsbedingung für eine fraktionale Ladung (FC) und eine Zwangsbedingung für einen fraktionalen Spin (FS) beinhalten.

11. Verfahren nach Anspruch 9 oder 10, wobei:
der Trainingsverlust ferner einen selbstkonsistenten Feldverlust (self-consistent field, SCF) beinhaltet, wobei der SCF-Verlust eine berechnete Energie des Atomsystems darstellt, die der Elektronenzahlerhaltung unterliegt.

12. Verfahren nach einem der Ansprüche 9 bis 11, ferner umfassend:
Erzeugen der ersten Teilmenge von Trainingsbeispielen (140, 240) durch numerische Synthese virtueller Atomsysteme mit elektronen-orbitalen Merkmalen und Energiestufen, die die eine oder die mehreren mathematischen Zwangsbedingungen erfüllen.

13. Verfahren nach einem der vorhergehenden Ansprüche, bei dem die elektronen-orbitalen Merkmale (110, 152) an jedem Gitterpunkt Merkmale einer Dichtematrix $\Gamma_{ab}^{\sigma}$ sind, die spinindiziert $\sigma \in \{\uparrow, \downarrow\}$ ist und auf einem Basissatz $\psi_a$ basiert, wobei der Basissatz aus Daten abgeleitet wurde, die eine molekulare Geometrie einer Vielzahl von Molekülen beschreiben, einschließlich Daten, die durch Messungen der realen Welt bestimmt werden.

14. System (100), umfassend:

einen oder mehrere Computer; und
eine oder mehrere Speichervorrichtungen, die Anweisungen speichern, die bei Ausführung durch den einen oder die mehreren Computer den einen oder die mehreren Computer dazu veranlassen, die Operationen des jeweiligen Verfahrens nach einem der Ansprüche 1 bis 13 durchzuführen.

15. Ein oder mehrere computerlesbare Speichermedien, die Anweisungen speichern, die beim Ausführen durch einen oder mehrere Computer den einen oder die mehreren Computer zum Durchführen der Operationen des jeweiligen Verfahrens nach einem der Ansprüche 1 bis 13 veranlassen.

**Revendications**

1. Procédé mis en œuvre par un ou plusieurs ordinateurs et permettant de prédire une énergie d'échange-corrélation (180, 224) d'un système atomique, le procédé comprenant :

l'obtention de caractéristiques respectives d'orbitales électroniques (110) du système atomique à chacun d'une pluralité de points de grille ;
la génération, pour chacun de la pluralité de points de grille, d'un vecteur de caractéristique d'entrée respectif (221) pour les caractéristiques d'orbitales électroniques (110) au point de grille ;
le traitement des vecteurs de caractéristique d'entrée respectifs (221) pour la pluralité de points de grille à l'aide d'un réseau neuronal (120) pour générer une énergie d'échange-corrélation prédite (180, 224) du système atomique ; et
dans lequel le réseau neuronal (120) est entraîné sur des données d'entraînement qui comportent une pluralité d'exemples d'entraînement (140, 150, 240, 250, 260), les exemples d'entraînement (140, 150, 240, 250, 260) correspondant chacun à un système atomique respectif, et les exemples d'entraînement (140, 150, 240, 250, 260) comportant un premier sous-ensemble d'exemples d'entraînement (140, 240) qui correspondent à des systèmes atomiques qui ont des caractéristiques d'orbitales électroniques et des niveaux d'énergie satisfaisant une ou plusieurs conditions de contrainte mathématique, les une ou plusieurs conditions de contrainte mathématique comportant : une condition de contrainte de gaz électronique uniforme (UEG), une condition de contrainte de charge fractionnaire (FC) ou une condition de contrainte de spin fractionnaire (FS) ; et soit :

une ou plusieurs des caractéristiques d'orbitales électroniques (110, 152) du système atomique sont obtenues sur la base de données de mesure du monde réel ; soit le procédé comprend en outre le fait de déterminer, sur la base de l'énergie d'échange-corrélation prédite (180, 224) du système atomique, s'il convient de réaliser un processus de fabrication d'un produit chimique qui comprend le système atomique et, si le fait de déterminer est positif, le fait d'amener la fabrication à être réalisée.

2.  Procédé selon la revendication 1, dans lequel :
    les systèmes atomiques correspondant à la pluralité d'exemples d'entraînement (140, 150, 240, 250, 260) comportent des systèmes atomiques virtuels générés synthétiquement avec des caractéristiques d'orbitales électroniques et des niveaux d'énergie satisfaisant les une ou plusieurs conditions de contrainte mathématique.

3.  Procédé selon l'une quelconque des revendications 1 ou 2, dans lequel les exemples d'entraînement (140, 150, 240, 250, 260) sont associés à des données décrivant des propriétés physiques du système atomique correspondant, les données d'une pluralité des exemples d'entraînement (150, 250) ayant été obtenues par des mesures réalisées dans le monde réel sur les systèmes atomiques correspondants.

4.  Procédé selon l'une quelconque des revendications 1 à 3, dans lequel :
    la pluralité de points de grille comporte des points de grille sur une grille en quadrature dans l'espace réel.

5.  Procédé selon l'une quelconque des revendications 1 à 4, dans lequel :
    les caractéristiques d'orbitales électroniques (110) obtenues pour le système atomique comportent un ou plusieurs des éléments suivants : une distribution de densité électronique, une distribution de norme de gradient de densité électronique, une distribution de densité d'énergie cinétique, une distribution d'échange Hartree-Fock (HF) locale ou une forme à portée séparée de la distribution d'échange HF locale du système atomique.

6.  Procédé selon l'une quelconque des revendications 1 à 5, dans lequel la génération du vecteur de caractéristique d'entrée (221) pour chacun de la pluralité de points de grille comporte :

    la conversion des caractéristiques d'orbitales électroniques (110) au niveau du point de grille d'une échelle linéaire à une échelle logarithmique ; et
    la concaténation des caractéristiques d'orbitales électroniques (110) à l'échelle logarithmique pour former le vecteur de caractéristique d'entrée (221) pour le point de grille.

7.  Procédé selon l'une quelconque des revendications 1 à 6, dans lequel le réseau neuronal (120) comporte :

    un perceptron multicouche (MLP) (122, 222) configuré pour traiter chacun des vecteurs de caractéristique d'entrée (221) à la pluralité de points de grille pour générer une pluralité de facteurs d'amélioration (223) caractérisant une pluralité de termes de contribution correspondant au vecteur de caractéristique d'entrée (221) ; et
    une couche en quadrature numérique (124) configurée pour intégrer une somme pondérée de la pluralité des termes de contribution mis à l'échelle par les facteurs d'amélioration (223) sur la pluralité de points de grille pour générer l'énergie d'échange-corrélation prédite (180, 224) du système atomique.

8.  Procédé selon la revendication 7, dans lequel :
    la pluralité de termes de contribution comporte un ou plusieurs des éléments suivants : un terme d'échange d'approximation de densité locale (LDA), un terme HF ou un terme HF à portée séparée.

9.  Procédé mis en œuvre par un ordinateur d'entraînement du réseau neuronal (120) selon l'une quelconque des revendications 1 à 8, le réseau neuronal (120) comportant une pluralité de paramètres (170), et le procédé comprenant :

    l'obtention d'une pluralité d'exemples d'entraînement (140, 150, 240, 250, 260), chaque exemple d'entraînement (140, 150, 240, 250, 260) comportant des caractéristiques d'orbitales électroniques (152) et une étiquette d'énergie réelle correspondante (154) d'un système atomique, la pluralité d'exemples d'entraînement (140, 150, 240, 250, 260) comportant un premier sous-ensemble d'exemples d'entraînement (140, 240) qui correspondent à des systèmes atomiques ayant des caractéristiques d'orbitales électroniques et des niveaux d'énergie satisfaisant une ou plusieurs conditions de contrainte mathématique, dans lequel les une ou plusieurs conditions de contrainte mathématique comportent : une condition de contrainte de gaz électronique uniforme (UEG), une condition de contrainte de charge fractionnaire (FC) ou une condition de contrainte de spin fractionnaire (FS) ; pour chaque exemple d'entraînement (140, 150, 240, 250, 260), le traitement des caractéristiques d'orbitales électroniques dans l'exemple d'entraînement (140, 150, 240, 250, 260) à l'aide du réseau neuronal (120) et conformément aux valeurs actuelles des paramètres (170) pour générer une énergie d'échange-corrélation prédite (180, 224) pour l'exemple d'entraînement (140, 150, 240, 250, 260) ;

la détermination d'un gradient par rapport aux paramètres (170) d'une perte d'entraînement, la perte d'entraînement comportant une perte de régression qui mesure, pour chaque exemple d'entraînement (140, 150, 240, 250, 260), une erreur entre l'énergie d'échange-corrélation prédite (180, 224) pour l'exemple d'entraînement et l'étiquette d'énergie réelle (154) dans l'exemple d'entraînement ; et

la mise à jour des valeurs actuelles des paramètres (170) à l'aide du gradient.

10. Procédé selon la revendication 9, dans lequel :
les une ou plusieurs conditions de contrainte mathématique comportent une condition de contrainte de charge fractionnaire (FC) et une condition de contrainte de spin fractionnaire (FS).

11. Procédé selon la revendication 9 ou 10, dans lequel :
la perte d'entraînement comporte en outre une perte de champ auto-cohérent (SCF), la perte SCF représentant une énergie calculée du système atomique soumis à la conservation du nombre d'électrons.

12. Procédé selon l'une quelconque des revendications 9 à 11, comprenant en outre :
la génération du premier sous-ensemble d'exemples d'entraînement (140, 240) en synthétisant numériquement des systèmes atomiques virtuels avec des caractéristiques d'orbitales électroniques et des niveaux d'énergie satisfaisant les une ou plusieurs conditions de contrainte mathématique.

13. Procédé selon une quelconque revendication précédente, dans lequel les caractéristiques d'orbitales électroniques (110, 152) à chaque point de grille sont des caractéristiques d'une matrice de densité $\Gamma^{\sigma}_{ab}$ indexée par le spin $\sigma \in \{\uparrow, \downarrow\}$ et sur la base d'un ensemble de base $\psi_a$, l'ensemble de base ayant été dérivé de données décrivant une géométrie moléculaire d'une pluralité de molécules, comportant des données déterminées par des mesures du monde réel.

14. Système (100) comprenant :

un ou plusieurs ordinateurs ; et
un ou plusieurs dispositifs de stockage stockant des instructions qui, lorsqu'elles sont exécutées par les un ou plusieurs ordinateurs, amènent les un ou plusieurs ordinateurs à réaliser les opérations du procédé respectif selon l'une quelconque des revendications 1 à 13.

15. Un ou plusieurs supports de stockage lisibles par ordinateur stockant des instructions qui, lorsqu'elles sont exécutées par un ou plusieurs ordinateurs, amènent les un ou plusieurs ordinateurs à réaliser les opérations du procédé respectif selon l'une quelconque des revendications 1 à 13.

Input data specifying orbital features 110

Exchange-correlation energy prediction system 100

Training example generation engine 130

Neural network 120

MLP 122

Numerical quadrature layer 124

Synthesized training examples satisfying exact conditions 140

Additional training examples 150

Orbital features 152

Energy labels 154

Training engine 160

Network parameters 170

Predicted exchange-correlation energy 180

FIG. 1

**FIG. 2**

300

Obtain electron-orbital features of an atomic system
<u>310</u>

Generate input feature vectors  <u>320</u>

Process the input feature vectors using a neural
network to generate  predicted exchange-correlation
energy  <u>330</u>

# FIG. 3

400

```
┌─────────────────────────────────────────────────┐
│         Obtain training examples 410             │
└─────────────────────────────────────────────────┘
                        │
                        ▼
┌─────────────────────────────────────────────────┐
│  Generate predicted exchange-correlation energy  │
│          for each training example   420         │
└─────────────────────────────────────────────────┘
                        │
                        ▼
┌─────────────────────────────────────────────────┐
│  Determine gradients of a training loss with     │
│  respect to the neural network parameters   430  │
└─────────────────────────────────────────────────┘
                        │
                        ▼
┌─────────────────────────────────────────────────┐
│  Update the current values of the neural network │
│              parameters  440                      │
└─────────────────────────────────────────────────┘
```

# FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63135223

**Non-patent literature cited in the description**

- **J. SCHMIDT et al.** *Machine Learning the Physical Local Exchange-Correlation Functional of Density-Functional theory*, 2019
- **R. NAGAI et al.** *Completing Density Functional Theory by Machine-learning hidden messages from molecules*, 2019
- **GOERIGK, L. et al.** A look at the density functional theory zoo with the advanced GMTKN55 database for general main group thermochemistry, kinetics and noncovalent interactions. *Phys. Chem. Chem. Phys.*, 2017, vol. 19, 32184-32215